# EUROPEAN PATENT SPECIFICATION

(11) **EP 3 146 997 B1**
(45) Date of publication and mention of the grant of the patent: **31.01.2018**
(21) Application number: 16158669.8
(22) Date of filing: 04.03.2016
(51) Int. Cl.: A61N 5/06, A61H 39/08, A61N 5/067, A61H 39/00, A61B 17/34, H01S 3/067, A61B 17/00

(54) **OPTICAL NEEDLE**
OPTISCHE NADEL
AIGUILLE OPTIQUE

(30) Priority: 24.09.2015 TW 104131666
(43) Date of publication of application: 29.03.2017
(73) Proprietor: Tseng, Hsiao-Sen, 42050 Taichung City (TW)
(72) Inventor: Tseng, Hsiao-Sen, 42050 Taichung City (TW)
(74) Representative: Patentship Patentanwaltsgesellschaft mbH

(56) References cited:
- WO-A1-2014/029423
- DE-U1- 20 007 456
- US-A1- 2004 010 204

## Description

### FIELD OF THE INVENTION

The present invention relates to an optical needle, more particularly to a puncture needle having light-guiding characteristics, in order to transmit a light beam into an object.

### BACKGROUND OF THE INVENTION

The puncture needle is a tool widely used in industry, services and daily life. In many applications, a puncture needle needs to have a light-guiding function. Such applications include industrial detection, medical detection and treatment.

Recently, stimulations by puncture needles at certain "acupoints" of the human body, in order to improve the physical conditions of a person have become a popular application in many countries. One of such applications is called "acupuncture", where a puncture needle is inserted into the human body through the skin, to reach the position of an acupoint, and thermal, optical or electrical energy is applied to the acupoint through the puncture needle, in order to stimulate physiological reactions at the acupoint. One way to apply the energy is to burn the external portion of the puncture needle, in order to transmit the thermal energy to the acupoint through the puncture needle. Another way is to apply an electricity to the needle, in order to transmit the electrical energy to the acupoint through the needle. A most recent application of the puncture needle is to apply a light beam, in particular a laser beam, to the acupoint through the puncture needle.

In order to transmit light beams through a puncture needle, many optical needles to be used in medical or acupuncture applications have been developed. In addition, optical needles for other applications based on transmission of light beams through a puncture needle, such as detections using the light beams so transmitted, have also been developed.

CN103901233A discloses a probe with an optical fiber. An end of the optical probe is etched to form a tip with an oval end surface. A metal coating is provided around the tip, with the tip exposed from the coating.

CN104287960A discloses an acupuncture needle with an optical fiber provided inside the acupuncture needle. An end of the optical fiber forms a tapered tip at a micro pore of the tip of the acupuncture needle, to guide a laser beam into a target position of the needle. Other micro pores are provided for the transmission of electrical and optical signals.

CN204073134 discloses a multi-channel laser treatment equipment, including eight mutually independent laser treatment channels, each including a laser light source and an optical fiber. Laser light is provided in the form of continuous or pulsed irradiations to irradiate an acupoint.

TW M493360U discloses an optical needle for intravenous irradiations. The optical needle provides a through hole at its base. An end of the through hole may be connected by a tube, to be inserted by an optical fiber, such that the optical fiber passes through the through hole. A cap is provided to seal the through hole, after the optical fiber is sterilized.

US 2014/0121538A1 discloses an assembly of an optical fiber and a metal needle, which provides a plurality of optical fiber tunnels therein. The tip of the needle forms two tilt angles so that an end of the optical fiber protruding from a second tilt angle, without protruding from the first tilt angle.

US 2014/0243806A1 discloses a hollow needle with optical fibers embedded therein. A plurality of tunnels is provided in the needle, to accommodate the plurality of optical fibers. A hub is provided to connect the plurality of optical fibers to a laser source. In the hub, a plurality of lightguides is provided, to guide laser beams from the laser source to the respective optical fibers.

WO 2014/133500A1 discloses a diagnostic probe. The probe includes a needle body provided with a plurality of tunnels to accommodate optical fibers. The respective optical fibers terminate at different longitudinal positions of the needle body, to collect diagnostic information of tissues surrounding the terminals.

Observations in the development of the optical needle reveal that certain puncture needles have been designed to provide a light-guiding function. These optical needles, however, used a complicated needle body structure. Most optical needles are connected to a separate light source through an optical fiber. In order to connect the lightguide embedded in the optical needle to the optical fiber that is capable of transmitting a light beam for a certain distance, a coupler to align the lightguide and the optical fiber will be necessary. The coupler makes the optical needle system bulky and adds additional costs to the manufacture and application of the system.

### OBJECTIVES OF THE INVENTION

The objective of the present invention is to provide a novel structure for the optical needle to enable the integration of the optical needle and a light source.

Another objective of the present invention is to provide an optical needle that does not need an optical coupler.

Another objective of the present invention is to provide an optical needle that is connectable to a light source.

Another objective of the invention is to provide a novel method for the preparation of an optical needle that is connectable to a light source.

### SUMMARY OF THE INVENTION

According to this invention, an optical needle is provided and comprises a needle body, a light source coupling seat and a lightguide. The needle body provides a cavity to accommodate a portion of the lightguide. The light source coupling seat provides a junction plane for interfacing a light source and the lightguide. The junction plane may be provided in a recess that can accommodate the light source. An end of the needle body is aligned with the junction plane of the light source coupling seat, such that an end of the lightguide is aligned with the junction plane or the light source. The other end of the lightguide terminates at the tip of the needle body. The optical needle is characterized in that the cross sectional area of the lightguide reduces from a light source section to a needle body section. In certain embodiments, the lightguide is an optical fiber. The reduction of the cross sectional area is a continuous reduction or a gradient reduction.

In a particular embodiment of the invention, the light source coupling seat extends in the radial direction of the lightguide, in order to facilitate insertion of the optical needle into an object. In application, the optical needle is used to transmit light beams into an object, which may be a human tissue. The contact surface of the light source coupling seat with the object may be a substantial plane. The junction plane may also be provided on a protruding portion, which may be engaged in a recess provided in a light source coupler. The tip of the optical needle may form a diagonal cut to facilitate insertion. The top surface of the optical needle may extend to form a plane, to strengthen the combination of the needle body and the light source coupling seat.

The optical needle of the present invention may further comprise a light source. The light source may be an optical fiber cable or a laser head. If the light source is a laser head, the laser head may comprise a laser beam generator for generating a laser beam; a power supply for providing power to the laser beam generator; a coupler to couple the light source to the coupling seat; and a switch for the control of the power supply. The laser head may provide a coupling portion with a protrusion having a shape complimentary to the shape of the recess of the light source coupling seat. The coupling portion may also have a recess with a shape complimentary to the shape of the protruding portion of the light source coupling seat. The protrusion and/or recess of the coupling portion facilitates stable insertion of the laser lead in the light source coupling seat and the alignment of the light emitting surface of the laser lead to the light source end of the lightguide.

The present invention also discloses a preparation method of optical needle. The method comprises: preparing a needle body with a hollow cavity; providing a lightguide with a first section, a narrowing section connected to the first section and a second section connected to the narrowing section. The cross sectional area of the lightguide reduces from a junction of the first and narrowing sections to a junction of the narrowing and second sections. The method further comprises: placing the second section of the lightguide in the hollow cavity; if necessary, leaving the first and/or narrowing section outside of the hollow cavity; forming a light source coupling seat with a light source junction plane, such that the light source coupling seat encloses a portion of the needle body and the first and narrowing sections of the lightguide and that a first end of the lightguide is aligned with the light source junction plane, to receive light beams entering through the light source junction plane. The light source junction plane may be provided in a light source accommodation cavity, such that, when the light source coupling seat is formed, the first end of the lightguide is aligned with the light source accommodation cavity. The light source junction plane may also be provided on a protruding portion of the light source coupling seat.

These and other objectives and advantages of present invention may be clearly appreciated from the detailed description by referring to the following drawings.

The invention is defined in the claims, other embodiments being merely exemplary.

### BRIEF DESCRIPTION OF THE DRAWINGS

Figure 1 shows the perspective view of one embodiment of the optical needle of the present invention, while Figure 1A is a schematic diagram of the optical needle of Figure 1, after assembly.
Figure 2 shows the cross-sectional schematic view of the optical needle of Figure 1, while Figure 2A is a schematic diagram of the optical needle of Figure 1, after assembly.
Figure 3 is the flowchart of the method for preparation of an optical needle in accordance with the present invention.
Figure 4 shows the exploded view of one example of a laser head applicable in the optical needle of the present invention, while Figure 4A is a schematic diagram of the laser head of Figure 4, after assembly.

### DETAILED DESCRIPTION OF THE INVENTION

The present invention provides a novel structure of optical needles and its preparation method. The new structure is connectable to a laser source. The present invention also provides an optical needle in combination with a laser source. Although it is not intended to limit the present invention by any theory, the inventor has found that one reason that makes the conventional optical needles complicated in structure is the power consumption of the light source. In order to supply light beams with sufficient power, the light source or laser source is made bulky. In addition, size of lightguide or optical fiber or optical fiber cable used in the optical needle is incompatible with that of the optical fiber used to transmit light beams for a relatively long distance. To connect the lightguide and the optical fiber/cable with high efficiency, a coupler is needed. The inventor also found that the commercially available, small-size laser heads are able to generate laser beams with a wavelength of about 405 nm to 660 nm. The laser beams so generated are sufficient to support most applications of the optical needle, where about 1mW or stronger light power is needed. In addition, by reducing the cross sectional area or diameter of a lightguide or an optical fiber along the longitudinal direction, the lightguide or optical fiber so obtained would be able to support the coupling of the optical fiber and a light source or a laser source. An invention with these and other features is thus realized.

In the followings, the optical needle of the present invention will be described by using its several embodiments. It shall be appreciated that description of the embodiments serves merely to illustrate the basic structure and spirit of the present invention. They shall not be used to limit the scope of protection of this invention.

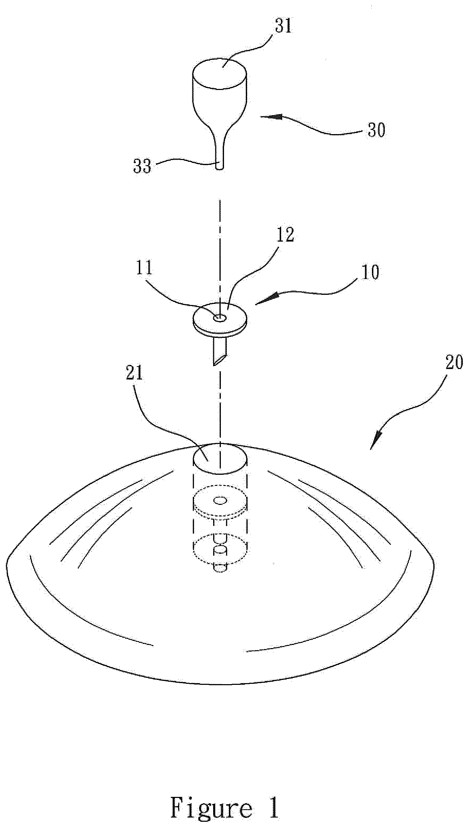

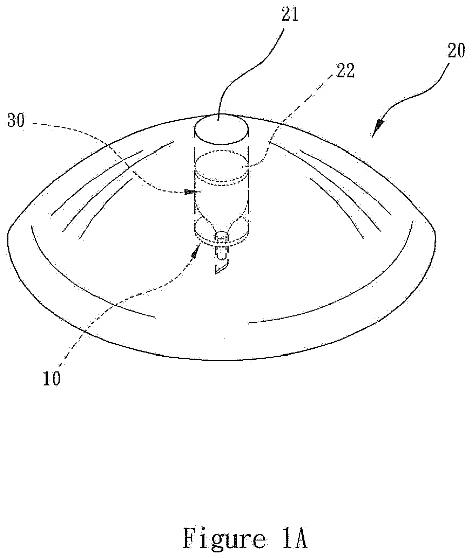

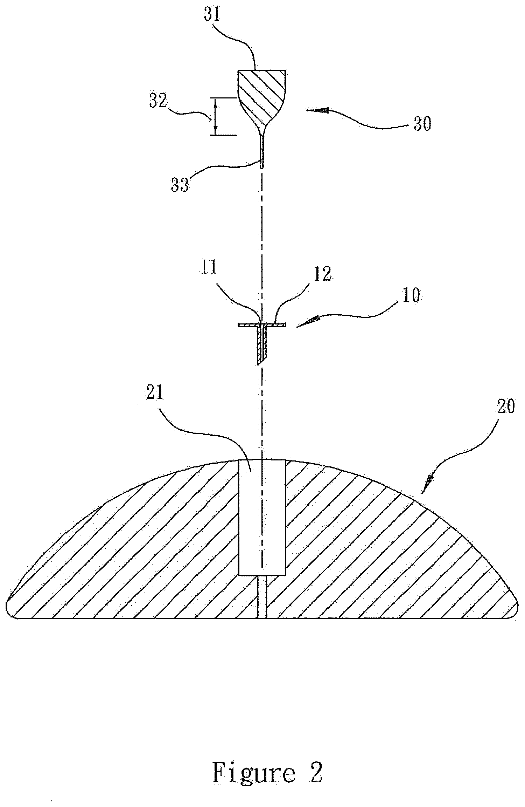

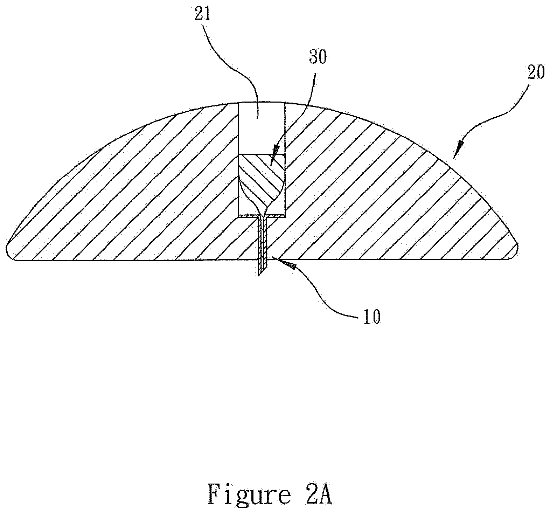

Figure 1 shows the perspective view of one embodiment of the optical needle of the present invention and Figure 2 shows its cross-sectional view. As shown, the optical needle of the present invention includes a needle body 10, a light source coupling seat 20 and a lightguide 30. The needle body 10 has a cavity 11 for receiving the second portion 33 of the lightguide 30, in this case an optical fiber.

In the preferred embodiment of the inventions, the needle body 10 may be made from any rigid material. Suitable materials include metal, plastics, glass, or carbon fiber. In the example of FIG. 1 and 2, the top surface of the needle body 10 extends in the lateral direction and forms a disc 12. The purpose of the disc shape is to strengthen the combination of the needle body 10 and the coupling seat 20. The inner diameter of the cavity 11 of the needle body 10 may be of a fixed value or reduce from its the top to its tip section. At this tip portion, the inner diameter of the cavity 11 may be about 50 to 500um, preferably about 100-250um, and more preferably about 200um.

In a preferred embodiment of the present invention, the tip of the needle body 10 may have a diagonal cut (not shown) to facilitate puncture purposes of the needle 10. The shape of the cut is not limited but is preferably a shape easy to produce.

The light source coupling seat 20 may be made from a rigid or flexible material. Suitable materials include plastic, silicone, resin and other plastic material. It is also possible to use a metal, ceramic and other materials that are easy to process, to prepare the light source coupling seat 20. In the example of FIG. 1 and 2, the top surface of the light source coupling seat 20 forms a spherical shape. This, however, is not any technical limitation. For example, the top surface of the coupling seat 20 may be flat, concave or in other shape. The top surface may form a particular pattern or design, by using any applicable technique. In the example of these figures, the coupling seat 20 extends laterally for the convenience of puncturing and inserting the optical needle into an object. This, of course, is not any technical limitation. The object may be a human tissue, such as the aforementioned acupoints of the human body. In such examples, the optical needle may serve as an acupuncture needle. The lower surface of the light source coupling seat 20 is preferably formed a substantial plane, so that the optical needle forms a relatively large contact surface with the object after insertion, to avoid displacement. The lower surface may be formed of other non-planar shape, provided with a pattern, or applied with additional materials.

In the example of Figure 1, the light source coupling seat 20 provides a recess 21 which is open to the top surface of the coupling seat 20, whereby a cavity to accommodate a light source 40 is formed. Shape of the opening of the recess 21 preferably complement a contour of the corresponding portion of the light source 40, so that the light source 40 can be securely received within the recess 21. After assembly, the optical needle provides a light source junction plane 22 in the light source coupling seat 20, at a position inside the recess 21 and above the first end 31 of the lightguide 30. The junction plane 22 is preferably flat. If the light source coupling seat 20 does not provide the recess 21, the light source junction plane 22 may be provided on the top surface of the light source coupling seat 20. Therefore, the junction plane 22 and the top surface of the light source coupling seat 20 form substantial flush. In addition, the light source coupling seat 20 may also provide a protruding portion (not shown), at a portion above the first end 31 of the lightguide 30, so that the protruding portion of the coupling seat 20 may be engaged in a recess (not shown) possibly provided in the light source 40. Contour of this protruding portion preferably complement shape of the recess of the light source.

Those having ordinary skills in the art may appreciate that the junction plane 22 provided in the light source coupling seat 20 is not necessarily a physical plane at the surface of an object. The junction plane 22 may be an imaginary plane. Moreover, the junction plane 22 may also be a top surface of the first end 31 of the lightguide 30.

The lightguide 30 may be any light guiding material and is preferably an optical fiber. Suitable materials for the lightguide 30 include: glass, plastic, metal oxides and the like. A protective film may be provided on the surface of the lightguide 30. There is no particularly limitation in the material of the protective film. The cross-sectional shape of the lightguide 30 may be elliptical, but may also be circular, square, or polygonal or in a figure-8 configuration. If necessary, the lightguide 30 can also be a beam with two or more optical fibers twisted together.

One of the features of the present invention is that the cross sectional area of the lightguide 30 reduces from its light source section (first section) 31 to its needle body end (second section) 33. When an optical fiber of circular cross-section is used, the diameter of the optical fiber reduces. Reduction of the cross sectional area may be a continuous reduction or a gradient reduction. Under this design, the lightguide 30 will include along its length direction: a first section 31, a narrowing section 32 connected to the first section 31, and a second section 33 connected to the narrowing section 32. The first section 31 is aligned with the recess 21 (Embodiment of FIG. 1) of the coupling seat 20, to be coupled to the light source 40. The second section 33 is to be disposed within the hollow cavity 11 of the needle body 10, and to extend to the tip portion of the needle body 10. Thecross sectional area, or diameter, of the lightguide 30 starts to reduce from the junction of the first section 31 and the narrowing section 32 and the reduction ends at the junction of the narrowing section 32 and the second section 33. In the application of the present invention, the diameter of the first section 31 may be between 200um to 1000um, preferably between 450um to 500um. The exact size of the first section 31 is not any technical limitation and is preferably compatible with the size of a light emitting surface of the light source 40. The diameter of the second section 33 may be between 30um to 100um, preferably between 40um to 50um. The exact size of the second section 33 is not any technical limitation and is preferably compatible with the size of an inner diameter of the cavity 11. If the inner diameter of the cavity 11 at the tip portion is 100 um, the diameter of the second section 33 may be 50um, so that the lightguide 30 may be easily disposed in the cavity 11 of the needle body 10.

The second section 33 preferably terminates at the needle tip, while it may retract within the needle tip or extend beyond the needle tip.

Method for forming the narrowing section 32 of the lightguide 30 is not limited but is preferably a technique to produce a gradual or progressive reduction in the cross sectional area of an elongated lightguide, such as an optical fiber. Suitable methods include heating stretch at high temperature, molding and other methods. Among them, stretching at high temperature produces an optical fiber with continuously reduced cross sectional area; the product is advantageous in transmission of light beams. Molding method forms a lightguide with gradient reduction in cross sectional area, the advantage of which is accuracy in size of each section. Length of the narrowing section 32 is not particularly limited, but is preferably as short as possible. For example, if the diameter of the first section 31 is 500um in length and the diameter of the second section 33 is 50um in length, length of the narrowing section 32 can be 1mm to 5mm, so that the reduction ratio is 1/10 to 1/2 per mm. This ratio can reduce the length of the narrowing section 32, while efficient transmission of optical power is obtained. Other reduction ratios can also be used in the present invention, to obtain the same or similar effects. In addition, the reduction ratio is not necessarily linear.

The structure of the needle body 10 is preferably designed to accommodate the narrowing section 32 in the cavity, when the optical fiber 30 is disposed within the needle 10. If the cavity 11 has a fixed inner diameter along its length direction, the narrowing section 32 and the first section 31 will be disposed external to the cavity 11, i.e., beyond the top end 12 of the needle body 10. On the other hand, if the inner diameter of the cavity 11 is progressive, i.e., the inner diameter reduces from the top end 12 to the tip direction, the narrowing section 32 or a portion thereof will be disposed in the cavity 11. After the lightguide 30 is assembled in the needle body 10, the top end 12 of the needle body 10 is aligned with the recess 21 of the light source coupling seat 20, such that end surface of the first section 31 is aligned with the light emitting surface of the light source 40 to be accommodated in the recess 21 of the coupling seat 20.

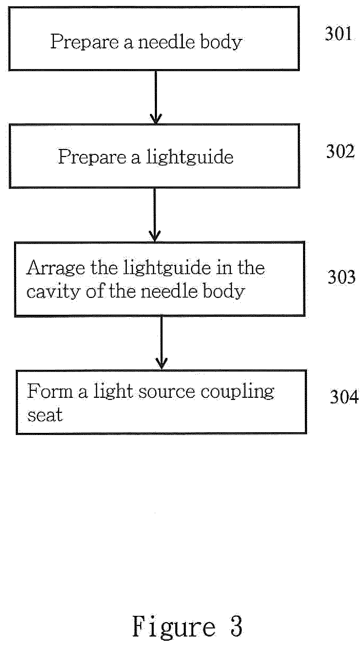

In the followings, a method for preparation of the optical needle of the present invention will be described. Figure 3 is the flowchart of the method for preparation of an optical needle in accordance with the present invention. As shown, in the preparation of the optical needle of the present invention, firstly at 301 a needle body 10 is prepared. The needle body 10 has a cavity 11, with a fixed or progressive diameter along its length direction. The top end of the needle body 10 may further include a disc 12 extended laterally. Secondly, at step 302 a lightguide 30, in this case an optical fiber, is prepared. The optical fiber 30 has a first section 31, a narrowing section 32 connected to the first section 31, and a second section 33 connected to narrowing section 32. The cross sectional area of the optical fiber 30 reduces from the junction of the first section 31 and the narrowing section 32 to the junction of the narrowing section 32 and the second section 33. The reduction may be a gradual reduction or a gradient reduction. Then, at 303 the second section 33of the optical fiber 30 is disposed in the cavity 11 of the needle body 10. If the inner diameter of the cavity 11 is fixed, the narrowing section 32 and/or the first section 31 remains outside of the cavity 11. At 304, a light source coupling seat 20 with a light source accommodation cavity 21 is formed, to encompass a portion of the needle body 100 and the first section 31 and narrowing section 32 of the lightguide 30, while having an end surface of the first section 31 aligned with the light source accommodation cavity 21, in order to receive light beams entering into the light source accommodation cavity 21. Preparation of the invented optical needle is thus completed.

In another embodiment of the present invention, the light source coupling seat 20 does not provide the light source accommodation cavity 21 but, instead, a light source junction plane 22 at the top surface of the coupling seat 20, or even on a protruding portion of the coupling seat 20. In such embodiments, since a narrowing section 32 that is useful in coupling the optical fiber 30 to an optical fiber cable has been provided, simply irradiating the junction plane 22 with a commercially available laser source, such as a laser pointer, would cause the transmission of light power to the second section 33 of the lightguide 30.

In the above steps, the light source coupling seat 20 is preferably formed by molding, such as injection molding. In such examples, the products of step 303 are arranged at suited positions in a mold for the light source coupling seat 20 and maintain their relative positions. A material for the light source coupling seat 20 is filled in the mold to form the coupling seat 20. After necessary annealing and other process, the optical needle of the present invention is prepared. In order to maintain the relative positions of the lightguide 30 and the needle body 10, certain positioning tools may be used. The tools will become part of the coupling seat 20 after formation of the coupling seat 20.

The optical needle of the present invention may further comprise a light source 40. The light source 40 may be an optical fiber cable or a laser head. If the light source 40 is an optical fiber cable, an end of the cable will be connected with a light source (not shown), to generate light power needed in the optical needle, whereby the light power may be transmitted to the optical needle through the cable. In such embodiments, the other end of the optical fiber cable may be inserted in the recess 21 of the coupling seat 20, whereby the narrowing section 32 of the lightguide 30 will couple the light power of the light source to the second section 33 for further use.

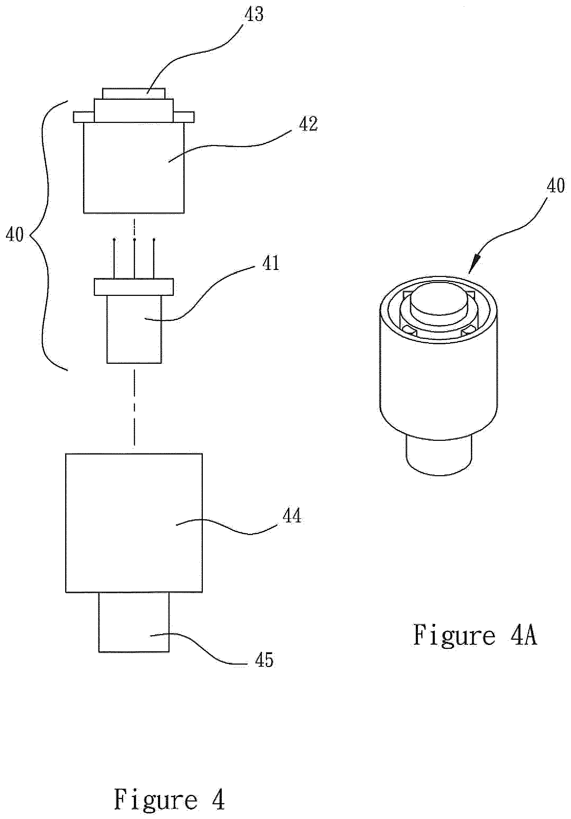

In a preferred embodiment of the present invention, the light source 40 is a laser head. Figure 4 shows the exploded view of one example of a laser head 40 applicable in the optical needle of the present invention, while Figure 4A is a schematic diagram of the laser head 40 of Figure 4, after assembly.

As shown, the laser head 40 includes a laser beam generator 41 for generating a laser beam; a power supply 42 for providing power to the laser beam generator 41; and a switch 43 to control the power supply 42. Figure 4 also shows that the laser beam generator 41, the power supply 42 and the switch 43 are assembled in a coupler 44 and integrated to form a laser head 40. The coupler 44 provides a coupling end 45, to function as a light emitting surface of the laser head 40. The coupler 44 may be inserted in the recess 21 of the light source coupling seat 20, such that the light emitting surface is aligned with end surface of the first section 31 of the lightguide 30. In this design, the shape and size of the coupling end 45 is preferably compatible with the recess 21, so that the laser head 40 can be inserted and securely placed in the light source coupling seat 20, whereby the light-emitting surface is aligned with end surface of the first section 31 of the lightguide 30.

Any small-scale laser light generator can be used in the present invention. For example, the commercially available laser pointer that generates red-color laser beams, is applicable in this invention. Laser light generated by such laser source has a wavelength of about 405nm to 660nm, which is sufficient to provide about 1mW or higher light power, sufficient for most applications of the optical needle. Since this and other laser sources are well known in the art, details thereof is omitted.

In addition, power supply 42 that supplies electrical power to such a laser source 41 may be any commercially available small-size battery. Micro switch 43 to control such power supply 42 is also known. The combination of the laser light generator 41, power supply 472 and switch 43 may be determined according to needs in particular applications. Details thereof is also omitted.

In addition to the types of light source described above, it is also possible to irradiate the junction plane 22 inside the recess 21 or at the top surface of the coupling seat 20 by a commercially available laser source, such as a laser pointer as described above, to achieve the transmission of light powers to the second section 33 of the lightguide 30. In other words, in application an optical fiber cable or a laser head inserted in the coupling seat 20 is not absolutely necessary.

As described above, the present invention provides a new structure for the optical needle. The optical needle is connectable with a variety type of light sources, without the need of an additional coupler. A compact and small-size optical needle that is able to reduce the foot print of the optical needle application system is thus provided.

## Claims

1. An optical needle, comprising a needle body (10), a light source coupling seat (20) and a lightguide (30); wherein the needle body provides a cavity (11) to accommodate a portion of the lightguide (30); the light source coupling seat (20) provides a junction plane (22) for interfacing a light source (40) and the lightguide (30); and an end of the needle body (10) is aligned with the junction plane (22) of the light source coupling seat (20), such that an end of the lightguide (30) is aligned with the junction plane (22); **characterized in that** the lightguide (30) comprises a first section (31), a narrowing section (32) connected to the first section (31) and a second section (33) connected to the narrowing section (32), and that a cross sectional area of the lightguide (30) reduces from a junction of the first (31) and narrowing (32) sections to a junction of the narrowing (32) and second (33) sections.

2. The optical needle according to claim 1, wherein the junction plane (22) is provided in a recess (21) that can accommodate the light source (40).

3. The optical needle according to claim 1, wherein the junction plane (22) is provided on a protruding portion, formed at a top surface of the light source coupling seat (20).

4. The optical needle according to claim 2, further comprising a laser head (40), said laser head (40) comprising: a laser beam generator (41) for generating a laser beam; a power supply (42) for providing power to the laser beam generator (41); a coupler (44) to couple the light source (40) to the coupling seat (20); and a switch (43) for the control of the power supply (42); wherein a coupling portion with a protrusion having a shape complimentary to a shape of the recess (21) of the light source coupling seat (20) is provided, such that a light emitting surface of the laser lead (40) is aligned to the light source end of the lightguide (30).

5. The optical needle according to anyone of claims 1-4, wherein the lightguide (30) comprises an optical fiber.

6. The optical needle according to anyone of claims 1-4, wherein reduction in cross sectional area of the lightguide (30) is a gradual reduction or a gradient reduction.

7. The optical needle according to claim 1, wherein the light source coupling seat (20) extends in the radial direction of the lightguide (30) and a lower surface of the light source coupling seat (20) forms a substantial plane.

8. The optical needle according to anyone of claims 1-4, wherein a tip of the optical needle forms a diagonal cut.

9. The optical needle according to anyone of claims 1-4, wherein the light source coupling seat (20) is prepared from a plastic material and encompasses a portion of the needle body (10) and a light source end of the lightguide (30).

10. A method for preparation of an optical needle, comprising the steps of:
preparing (301) a needle body (10) with a hollow cavity (11);
providing (302) a lightguide (30) with a first section (31), a narrowing section (32) connected to the first section (31) and a second section (33) connected to the narrowing section (32); wherein a cross sectional area of the lightguide (30) reduces from a junction of the first (31) and narrowing sections (32) to a junction of the narrowing (32) and second sections (33);
disposing (303) the second section (33) of the lightguide (30) in the hollow cavity (11); and
forming (304) a light source coupling seat (20) with a light source junction plan (22), such that the light source coupling seat (20) encompasses a portion of the needle body (10) and the first section (31) of the lightguide (30) and that a first end of the lightguide (30) is aligned with the light source junction plane (22), to receive light beams entering through the light source junction plane (22).

11. The method according to claim 10, wherein the lightguide (30) comprises an optical fiber.

12. The method according to claim 10 or 11, wherein reduction in cross sectional area of the lightguide (30) is a gradual reduction or a gradient reduction.

13. The method according to claim 10 or 11, wherein the light source coupling seat (20) extends in the radial direction of the lightguide (30) and a lower surface of the light source coupling seat (20) forms a substantial plane.

14. The method according to claim 10 or 11, wherein a tip of the optical needle forms a diagonal cut.

15. The method according to claim 10 or 11, wherein the light source coupling seat (20) is prepared from a plastic material and encompasses a portion of the needle body (10) and a light source end of the lightguide (30).

## Patentansprüche

1. Optische Nadel, umfassend einen Nadelkörper (10), einen Lichtquellen-Kopplungssitz (20) und einen Lichtleiter (30); wobei der Nadelkörper eine Kavität (11) bereitstellt, um einen Abschnitt des Lichtleiters (30) aufzunehmen; der Lichtquellen-Kopplungssitz (20) eine Verbindungsebene (22) zum Anschließen einer Lichtquelle (40) und des Lichtleiters (30) bereitstellt; und ein Ende des Nadelkörpers (10) an der Verbindungsebene (22) des Lichtquellen-Kopplungssitzes (20) ausgerichtet ist, so dass ein Ende des Lichtleiters (30) mit der Verbindungsebene (22) ausgerichtet ist; **dadurch gekennzeichnet, dass** der Lichtleiter (30) einen ersten Abschnitt (31), einen mit dem ersten Abschnitt (31) verbundenen sich verengenden Abschnitt (32) und einen mit dem sich verengenden Abschnitt (32) verbundenen zweiten Abschnitt (33) umfasst und dass sich eine Querschnittsfläche des Lichtleiters (30) von einer Verbindungsstelle des ersten (31) und des sich verengenden (32) Abschnitts zu einer Verbindungsstelle des sich verengenden (32) und des zweiten (33) Abschnitts verringert.

2. Optische Nadel nach Anspruch 1, wobei die Verbindungsebene (22) in einer Vertiefung (21) bereitgestellt ist, die die Lichtquelle (40) aufnehmen kann.

3. Optische Nadel nach Anspruch 1, wobei die Verbindungsebene (22) auf einem vorstehenden Abschnitt bereitgestellt ist, der auf einer oberen Fläche des Lichtquellen-Kopplungssitzes (20) gebildet ist.

4. Optische Nadel nach Anspruch 2, ferner umfassend einen Laserkopf (40), wobei der Laserkopf (40) umfasst: einen Laserstrahlerzeuger (41) zum Erzeugen eines Laserstrahls; eine Leistungsversorgung (42) zum Bereitstellen von Leistung für den Laserstrahlerzeuger (41); einen Koppler (44) zum Koppeln der Lichtquelle (40) mit dem Kopplungssitz (20); und einen Schalter (43) für die Steuerung der Leistungsversorgung (42); wobei ein Kopplungsabschnitt mit einem Vorsprung mit einer Form, die einer Form der Vertiefung (21) des Lichtquellen-Kopplungssitzes (20) komplementär ist, bereitgestellt ist, so dass eine lichtemittierende Fläche des Laserkopfes (40) am Lichtquellenende des Lichtleiters (30) ausgerichtet ist.

5. Optische Nadel nach einem der Ansprüche 1-4, wobei der Lichtleiter (30) eine optische Faser umfasst.

6. Optische Nadel nach einem der Ansprüche 1-4, wobei die Verringerung der Querschnittsfläche des Lichtleiters (30) eine allmähliche Verringerung oder eine abgestufte Verringerung ist.

7. Optische Nadel nach Anspruch 1, wobei sich der Lichtquellen-Kopplungssitz (20) in der radialen Richtung des Lichtleiters (30) erstreckt und eine untere Fläche des Lichtquellen-Kopplungssitzes (20) eine wesentliche Ebene bildet.

8. Optische Nadel nach einem der Ansprüche 1-4, wobei eine Spitze der optischen Nadel einen Schrägschnitt bildet.

9. Optische Nadel nach einem der Ansprüche 1-4, wobei der Lichtquellen-Kopplungssitz (20) aus einem Kunststoff hergestellt ist und einen Abschnitt des Nadelkörpers (10) und ein Lichtquellenende des Lichtleiters (30) umschließt.

10. Verfahren zur Herstellung einer optischen Nadel, umfassend die Schritte:
Herstellen (301) eines Nadelkörpers (10) mit einer hohlen Kavität (11);
Bereitstellen (302) eines Lichtleiters (30) mit einem ersten Abschnitt (31), einem mit dem ersten Abschnitt (31) verbundenen sich verengenden Abschnitt (32) und einem mit dem sich verengenden Abschnitt (32) verbundenen zweiten Abschnitt (33); wobei sich eine Querschnittsfläche des Lichtleiters (30) von einer Verbindungsstelle des ersten (31) und des sich verengenden Abschnitts (32) zu einer Verbindungsstelle des sich verengenden (32) und des zweiten Abschnitts (33) verringert;
Anordnen (303) des zweiten Abschnitts (33) des Lichtleiters (30) in der hohlen Kavität (11); und
Bilden (304) eines Lichtquellen-Kopplungssitzes (20) mit einer Lichtquellen-Verbindungsebene (22), so dass der Lichtquellen-Kopplungssitz (20) einen Abschnitt des Nadelkörpers (10) und des ersten Abschnitts (31) des Lichtleiters (30) umschließt und ein erstes Ende des Lichtleiters (30) an der Lichtquellen-Verbindungsebene (22) ausgerichtet ist, um Lichtstrahlen zu empfangen, die durch die Lichtquellen-Verbindungsebene (22) eintreten.

11. Verfahren nach Anspruch 10, wobei der Lichtleiter (30) eine optische Faser umfasst.

12. Verfahren nach Anspruch 10 oder 11, wobei die Verringerung der Querschnittsfläche des Lichtleiters (30) eine allmähliche Verringerung oder eine abgestufte Verringerung ist.

13. Verfahren nach Anspruch 10 oder 11, wobei sich der Lichtquellen-Kopplungssitz (20) in der radialen Richtung des Lichtleiters (30) erstreckt und eine untere Fläche des Lichtquellen-Kopplungssitzes (20) eine wesentliche Ebene bildet.

14. Verfahren nach Anspruch 10 oder 11, wobei eine Spitze der optischen Nadel einen Schrägschnitt bildet.

15. Verfahren nach Anspruch 10 oder 11, wobei der Lichtquellen-Kopplungssitz (20) aus einem Kunststoff hergestellt ist und einen Abschnitt des Nadelkörpers (10) und ein Lichtquellenende des Lichtleiters (30) umschließt.

## Revendications

1. Aiguille optique, comprenant un corps d'aiguille (10), un siège de couplage de source de lumière (20) et un guide lumière (30) ; dans laquelle le corps d'aiguille fournit une cavité (11) pour loger une portion du guide lumière (30) ; le siège de couplage de source de lumière (20) fournit un plan de jonction (22) pour servir d'interface entre une source de lumière (40) et le guide lumière (30) ; et une extrémité du corps d'aiguille (10) est alignée avec le plan de jonction (22) du siège de couplage de source de lumière (20), de sorte qu'une extrémité du guide lumière (30) soit alignée avec le plan de jonction (22) ; **caractérisée en ce que** le guide lumière (30) comprend une première section (31), une section de rétrécissement (32) raccordée à la première section (31) et une seconde section (33) raccordée à la section de rétrécissement (32), et **en ce qu'**une aire en coupe du guide lumière (30) diminue depuis une jonction de la première section (31) et de la section de rétrécissement (32) jusqu'à une jonction de la section de rétrécissement (32) et de la seconde section (33).

2. Aiguille optique selon la revendication 1, dans laquelle le plan de jonction (22) est fourni dans un évidement (21) qui peut loger la source de lumière (40).

3. Aiguille optique selon la revendication 1, dans laquelle le plan de jonction (22) est fourni sur une portion saillante, formée au niveau d'une surface haute du siège de couplage de source de lumière (20).

4. Aiguille optique selon la revendication 2, comprenant en outre une tête laser (40), ladite tête laser (40) comprenant : un générateur de faisceau laser (41) pour générer un faisceau laser ; une alimentation électrique (42) pour alimenter électriquement le générateur de faisceau laser (41) ; un coupleur (44) pour coupler la source de lumière (40) au siège de couplage (20) ; et un commutateur (43) pour la commande de l'alimentation électrique (42) ; dans laquelle une portion de couplage dotée d'une saillie ayant une forme complémentaire d'une forme de l'évidement (21) du siège de couplage de source de lumière (20) est fournie, de sorte qu'une surface d'émission de lumière de la tête laser (40) soit alignée avec l'extrémité de source de lumière du guide lumière (30).

5. Aiguille optique selon l'une quelconque des revendications 1 à 4, dans laquelle le guide lumière (30) comprend une fibre optique.

6. Aiguille optique selon l'une quelconque des revendications 1 à 4, dans laquelle une réduction d'aire en coupe du guide lumière (30) est une réduction progressive ou une réduction en gradient.

7. Aiguille optique selon la revendication 1, dans laquelle le siège de couplage de source de lumière (20) s'étend dans la direction radiale du guide lumière (30) et une surface inférieure du siège de couplage de source de lumière (20) forme un plan notable.

8. Aiguille optique selon l'une quelconque des revendications 1 à 4, dans laquelle une pointe de l'aiguille optique forme une coupe diagonale.

9. Aiguille optique selon l'une quelconque des revendications 1 à 4, dans laquelle le siège de couplage de source de lumière (20) est préparé à partir d'un matériau plastique et englobe une portion du corps d'aiguille (10) et une extrémité de source de lumière du guide lumière (30).

10. Procédé de préparation d'une aiguille optique, comprenant les étapes de :
préparation (301) d'un corps d'aiguille (10) doté d'une cavité creuse (11) ;
fourniture (302) d'un guide lumière (30) doté d'une première section (31), d'une section de rétrécissement (32) raccordée à la première section (31) et d'une seconde section (33) raccordée à la section de rétrécissement (32) ; dans lequel une aire en coupe du guide lumière (30) diminue depuis une jonction de la première section (31) et de la section de rétrécissement (32) jusqu'à une jonction de la section de rétrécissement (32) et de la seconde section (33) ;
disposition (303) de la seconde section (33) du guide lumière (30) dans la cavité creuse (11) ; et
formation (304) d'un siège de couplage de source de lumière (20) avec un plan de jonction de source de lumière (22), de sorte que le siège de couplage de source de lumière (20) englobe une portion du corps d'aiguille (10) et la première section (31) du guide lumière (30) et de sorte qu'une première extrémité du guide lumière (30) soit alignée avec le plan de jonction de source de lumière (22), pour recevoir des faisceaux de lumière entrant à travers le plan de jonction de source de lumière (22).

11. Procédé selon la revendication 10, dans lequel le guide lumière (30) comprend une fibre optique.

12. Procédé selon la revendication 10 ou 11, dans lequel une réduction d'aire en coupe du guide lumière (30) est une réduction progressive ou une réduction en gradient.

13. Procédé selon la revendication 10 ou 11, dans lequel le siège de couplage de source de lumière (20) s'étend dans la direction radiale du guide lumière (30) et une surface inférieure du siège de couplage de source de lumière (20) forme un plan notable.

14. Procédé selon la revendication 10 ou 11, dans lequel une pointe de l'aiguille optique forme une coupe diagonale.

15. Procédé selon la revendication 10 ou 11, dans lequel le siège de couplage de source de lumière (20) est préparé à partir d'un matériau plastique et englobe une portion du corps d'aiguille (10) et une extrémité de source de lumière du guide lumière (30).
